# EUROPEAN PATENT APPLICATION

(11) **EP 2 579 283 A1**
(43) Date of publication of application: **10.04.2013**
(21) Application number: 11789586.2
(22) Date of filing: 10.05.2011
(51) Int. Cl.: H01F 41/00, H01F 27/00

(54) **DIAGNOSIS METHOD AND DIAGNOSIS APPARATUS FOR OIL-FILLED ELECTRICAL APPARATUS**

(30) Priority: 25.03.2011 WO PCT/JP2011/057388; 02.06.2010 JP 2010127031
(71) Applicant: Mitsubishi Electric Corporation, Tokyo 100-8310 (JP)
(72) Inventor: TOYAMA, Satoru, Tokyo 100-8310 (JP); TANIMURA, Junji, Tokyo 100-8310 (JP); KATO, Fukutaro, Tokyo 100-8310 (JP); AMIMOTO, Tsuyoshi, Tokyo 100-8310 (JP); NAGAO, Eiichi, Tokyo 100-8310 (JP); KAWASHIMA, Takeshi, Tokyo 100-8310 (JP); HOSOKAWA, Noboru, Tokyo 100-8310 (JP)
(74) Representative: Sajda, Wolf E.
(86) International application number: PCT/JP2011/060768
(87) International publication number: WO 2011/152177

(57) **Abstract**

Provided is a diagnosis method for an oil-filled electrical apparatus (1), which is for evaluating the degree of danger of copper sulfide being generated within the oil-filled electrical apparatus (1), comprising: a first step for detecting a specific compound contained within insulating oil (55) inside the oil-filled electrical apparatus (1); a second step for evaluating the possibility of copper sulfide being generated inside the oil-filled electrical apparatus (1), on the basis of the result detected by the first step; and a third step for diagnosing the degree of danger of a malfunction occurring in the oil-filled electrical apparatus (1), on the basis of the evaluation result obtained in the second step. The specific compound contains dibenzyl disulfide and/or a reaction product of a radical resulting from dibenzyl disulfide, and
di-tert-butyl-p-cresol and/or a reaction product of a radical resulting from di-tert-butyl-p-cresol, or, di-tert-butyl-phenol and/or a reaction product of a radical resulting from di-tert-butyl-phenol.

## Description

### TECHNICAL FIELD

The present invention relates to anomaly diagnosis as to sulfidation corrosion of a transformer, and in particular, it relates to a technique of predicting generation of copper sulfide onto insulating paper.

### BACKGROUND ART

Sulfidation corrosion is such a phenomenon that conductive copper sulfide is generated by a reaction between copper which is the material for a transformer and a sulfur component in insulating oil. This copper sulfide is a semiconductor, and hence insulating performance lowers when the same adheres to an insulator.

Insulating paper is used for coil insulation particularly in a large-sized transformer or the like, and hence a short-circuit takes place between coils when copper sulfide adheres to this insulating paper which is an insulator, and the transformer is broken. However, the details of the generation mechanism thereof are unknown, and there has existed no effective diagnosis technique for an existing transformer.

The inventors have recently diligently studied a reaction mechanism related to copper sulfide generation, to recognize that such a reaction that dibenzyl disulfide adsorbs to a copper plate takes place as a first stage, such a reaction that the dibenzyl disulfide reacts with copper to generate a dibenzyl disulfide-Cu complex takes place as a second stage, and such a reaction that the dibenzyl disulfide-Cu complex decomposes into a benzyl radical and a benzyl sulfenyl radical and copper sulfide takes place as a third stage (NPL 1: S. Toyama, J. Tanimura, N. Yamada, E. Nagao, T. Amimoto, "Highly Sensitive Detection Method of Dibenzyl Disulfide and the Elucidation of the Mechanism of Copper Sulfide Generation in Insulating Oil", April 2009, IEEE TDEI Vol. 16, No. 2, pp. 509 to 515).

When using the technique of NPL 1, insulating oil can be collected from an existing transformer in operation, and the quantity of copper sulfide generated in the existing transformer can be predicted. A benzyl radical and a benzyl sulfenyl radical react with each other respectively and mutually, and bibenzyl, dibenzyl sulfide and dibenzyl disulfide generate as byproducts. Information related to generation of copper sulfide can be obtained by analysis of these byproducts.

However, whether or not adhesion of copper sulfide reaches an insulating paper has not been predictable even with this technique (PTL 1: Japanese Patent Laid-Open No. JP-A-2010-10439).

There is absolutely no technique of predicting generation of copper sulfide except for the aforementioned technique, and an operation of reducing a risk of copper sulfide generation is generally performed by analyzing dibenzyl disulfide which is a causative agent of copper sulfide by analysis of oil, collecting insulating oil in an existing transformer when this compound is detected and exchanging the same for new insulating oil or removing the dibenzyl disulfide by treating the collected insulating oil and thereafter returning the treated insulating oil into the transformer (PTL 2: U.S. Patent Laid-Open No. US-A- 2008/0251424).

There are a large number of techniques predicting inconvenience of a transformer other than generation of copper sulfide. Further, there are a large number of methods diagnosing abnormal overheat or abnormal discharge by detecting a certain type of compound. According to these methods, it is assumed that soundness of a transformer can be diagnosed by analyzing a volatile component in oil generated due to deterioration of insulating paper or the like.

For example, there is known an anomaly diagnosis method in an oil-filled electrical apparatus characterized in diagnosing abnormal overheat or abnormal discharge in the apparatus through the presence or absence of detection of acetic acid, 3-pentanone, 2,5-dimethylfuran, butyl aldehyde, 2-methoxy ethanol, methanethiol, dimethyl sulfide, ammonia, 1,3-diazine, methyl vinyl acetylene or 2-methyl-1,3-butadiene not detected from an ordinary oil-filled electrical apparatus in operation but easily detectable only in overheat or abnormal discharge (PTL 3: Claim 2 etc. of Japanese Patent Laid-Open No. JP-A-9-72 892). There are many cases where an anomaly of an oil-filled electrical apparatus results from dielectric breakdown of a coil or the like caused by adhesion of copper sulfide to insulating paper, and hence it is particularly important to predict adhesion of copper sulfide to an insulating paper surface in diagnosis of the oil-filled electrical apparatus. However, although the quantity or the like of copper sulfide generated in the oil-filled electrical apparatus can be predicted when using the invention of NPL 1 or PTL 1, whether or not the generated copper sulfide adheres to insulating paper cannot be determined.

Although the invention of PTL 2 is also a technique related to copper sulfide generation in a transformer, the same is not a technique allowing determination as to whether or not copper sulfide is generated on an insulating paper surface. Although the invention of PTL 3 is a technique of diagnosing an anomaly of a transformer, the same does not allow determination of generation of copper sulfide.

### CITATION LIST

### PATENT LITERATURE

| | |
|---|---|
| PTL 1: | Japanese Patent Laid-Open No. JP-A-2010-10 439 |
| PTL 2: | U.S. Patent Laid-Open No. US-A-2008/0251424 |
| PTL 3: | Japanese Patent Laid-Open No. JP-A-9-72 892 |

### NON PATEN LITERATURE

| | |
|---|---|
| NPL 1: | S. Toyama, J. Tanimura, N. Yamada, E. Nagao, T. Amimoto, "Highly Sensitive Detection Method of Dibenzyl Disulfide and the Elucidation of the Mechanism of Copper Sulfide Generation in Insulating Oil", April 2009, IEEE TDEI Vol. 16, No. 2, pp. 509 to 515. |

### SUMMARY OF THE INVENTION

### TECHNICAL PROBLEM

The present invention has been proposed in order to solve the aforementioned problems, and aims at providing a diagnosis method for an oil-filled electrical apparatus capable of determining whether or not copper sulfide is generable on an insulating paper surface of a coil or the like dipped in insulating oil in the oil-filled electrical apparatus.

### SOLUTION TO THE PROBLEM

The present invention provides a diagnosis method for an oil-filled electrical apparatus evaluating a risk of copper sulfide generation in the oil-filled electrical apparatus, including:
a first step of detecting a specific compound contained in insulating oil in the oil-filled electrical apparatus,
a second step of evaluating a possibility of copper sulfide generation in the oil-filled electrical apparatus on the basis of the result of detection obtained in the first step, and
a third step of diagnosing a risk of anomaly occurrence in the oil-filled electrical apparatus on the basis of the result of evaluation obtained in the second step, in which
the specific compound contains dibenzyl disulfide and/or a reaction product of a radical resulting from dibenzyl disulfide, and
di-tert-butyl-p-cresol and/or a reaction product of a radical resulting from di-tert-butyl-p-cresol, or, di-tert-butyl-phenol and/or a reaction product of a radical resulting from di-tert-butyl-phenol.

Preferably, the specific compound contains dibenzyl sulfide, and di-tert-butyl-p-cresol or di-tert-butyl-phenol.

Preferably, the reaction product of the radical resulting from the dibenzyl disulfide is a compound of at least one type selected from a group consisting of benzaldehyde, benzyl alcohol, bibenzyl, dibenzyl sulfide and dibenzyl sulfoxide.

Preferably, the specific compound contains a reaction product of a radical resulting from dibenzyl disulfide and a radical resulting from di-tert-butyl-p-cresol, or, a reaction product of a radical resulting from dibenzyl disulfide and a radical resulting from di-tert-butyl-phenol.

Preferably, the method evaluates in the second step that the possibility of copper sulfide generation in the oil-filled electrical apparatus is high in a case where the specific compound is detected in the first step, and
diagnoses in the third step that the risk of anomaly occurrence in the oil-filled electrical apparatus is high in a case where the possibility of copper sulfide generation is evaluated as high in the second step.

Preferably, copper sulfide generation in the oil-filled electrical apparatus is copper sulfide generation on a surface of insulating paper.

The present invention also relates to a diagnosis apparatus for an oil-filled electrical apparatus for evaluating a risk of copper sulfide generation in the oil-filled electrical apparatus, including:
a detection unit detecting a specific compound contained in insulating oil in the oil-filled electrical apparatus,
an evaluation unit evaluating a possibility of copper sulfide generation in the oil-filled electrical apparatus on the basis of the result of detection obtained in the detection unit, and
a diagnosis unit diagnosing a risk of anomaly occurrence in the oil-filled electrical apparatus on the basis of the result of evaluation obtained in the evaluation unit, in which
the specific compound contains dibenzyl disulfide and/or a reaction product of a radical resulting from dibenzyl disulfide, and
di-tert-butyl-p-cresol and/or a reaction product of a radical resulting from di-tert-butyl-p-cresol, or, di-tert-htztyl-phenol and/or a reaction product of a radical resulting from di-tert-butyl-phenol.

### ADVANTAGEOUS EFFECTS OF THE INTENTION

According to the inventive diagnosis method, such a nonconventional remarkable effect is attained that a risk of copper sulfide generation on an insulating paper surface of a coil or the like dipped in insulating oil in an oil-filled electrical apparatus can be correctly evaluated by detecting (measuring) not only dibenzyl disulfide but also di-tert-butyl-p-cresol or di-tert-butyl-phenol in analysis of insulating oil collected from an existing oil-filled electrical apparatus (transformer or the like).

### BRIEF DESCRIPTION OF DRAWINGS

- FIG. 1: is a graph showing a correlation between the quantities of reduction of DBDS and the total quantities of reaction products of radicals resulting from DBDS.
- FIG. 2A: is an example of a radical resulting from DBPC.
- FIG. 2B: is another example of a radical resulting from DBPC.
- FIG. 3: is an example of a compound having a molecular weight of 310 resulting from a reaction between a benzyl radical and DBPC.
- FIG. 4: is a block diagram of a diagnosis apparatus for an oil-filled electrical apparatus according to a fourth embodiment.
- FIG. 5: is a sectional view showing a structural example of the oil-filled electrical apparatus shown in FIG. 4.
- FIG. 6: is a plan view showing one of a plurality of winding layers constituting a coil.
- FIG. 7: is a sectional view showing a section of the wiring layer shown in FIG. 6 along the line A-A.
- FIG. 8: is a graph showing test results of a Test Example 3.

### DESCRIPTION OF EMBODIMENTS

As hereinabove described, it is recognized that such a reaction that dibenzyl disulfide (DBDS) adsorbs to a copper plate takes place as a first stage, such a reaction that DBDS reacts with copper to generate a DBDS-Cu complex takes place as a second stage, and such a reaction that the DBDS-Cu complex decomposes into a benzyl radical and a benzyl sulfenyl radical and copper sulfide takes place as a third stage as the mechanism of copper sulfide generation.

Among these compounds participating in the copper sulfide generation mechanism, only the copper sulfide is insoluble in insulating oil. When copper sulfide is generated on a copper plate surface, therefore, there is no possibility that this copper sulfide transfers to insulating paper.

On the other hand, the DBDS-Cu complex exhibits oil solubility since the same is a complex, and hence the same transfers from the copper plate surface to the insulating paper, and can be adsorbed to the insulating paper surface. In a case where such a reaction takes place that this DBDS-Cu complex decomposes into a benzyl radical and a benzyl sulfenyl radical and copper sulfide on the insulating paper surface, copper sulfide is generated on the insulating paper surface.

Further, when another radical is present in the vicinity of a sulfur element in such an instant that the DBDS-Cu complex radically decomposes on the copper plate surface, it is predicted that the sulfur element and another radical radically combine with each other to return to the complex.

Thus, the quantity of the copper sulfide generated on the copper plate surface decreases and the quantity of the oil-soluble complex increases, and hence it is conceivable that the quantity of generation of the copper sulfide on the insulating paper surface increases. In a case where another radical other than a radical deriving from DBDS coexists in insulating oil, therefore, it is predicted that the quantity of generation of copper sulfide on an insulating paper surface increases.

It is necessary that such another radical is present in the vicinity of the sulfur element stably and in a large quantity, and a radical-based antioxidant such as di-tert-butyl-p-cresol or di-tert-butyl-phenol (DBP) is conceivable, for example. The diagnosis method according to the present invention is characterized in detecting di-tert-butyl-p-cresol and/or a reaction product of a radical resulting from di-tert-butyl-p-cresol (or, di-tert-butyl-phenol and/or a reaction product of a radical resulting from di-tert-butyl-phenol). In the present invention, di-tert-butyl-p-cresol is preferably 2,6-di-tert-butyl-p-cresol (DBPC).

DBDS, DBPC and DBP are detectable according to an existing technique. The same can be determined up to 0.1 ppmw when employing a gas chromatograph/mass spectrometer, for example.

### First Embodiment

An embodiment of the diagnosis method for an oil-filled electrical apparatus according to the present invention is described. The diagnosis method according to this embodiment is a diagnosis method for an oil-filled electrical apparatus evaluating a risk of copper sulfide generation on an insulating paper surface of a coil or the like in the oil-filled electrical apparatus, and includes:
a first step of detecting a specific compound contained in insulating oil in the oil-filled electrical apparatus,
a second step of evaluating a possibility of copper sulfide generation in the oil-filled electrical apparatus on the basis of the result of detection obtained in the first step, and
a third step of diagnosing a risk of anomaly occurrence in the oil-filled electrical apparatus on the basis of the result of evaluation obtained in the second step.

More specifically, the possibility of copper sulfide generation in the oil-filled electrical apparatus is evaluated as high in the second step in a case where the specific compound is detected in the first step. In the third step, it is diagnosed that the risk of anomaly occurrence in the oil-filled electrical apparatus is high in a case where the possibility of copper sulfide generation is evaluated as high in the second step.

In this embodiment, DBPC and DBDS are detected (measured) as specific compounds.

According to this embodiment, it becomes possible to correctly evaluate a risk of copper sulfide generation on an insulating paper surface of a coil or the like dipped in insulating oil in an oil-filled electrical apparatus by detecting (measuring) not only DBDS but also DBPC in an analysis of insulating oil collected from an existing oil-filled electrical apparatus (transformer or the like).

### Second Embodiment

This embodiment is a diagnosis method different from the first embodiment in a point of measuring DBPC and a reaction product of a radical resulting from DBDS as the aforementioned specific compounds, and the remaining points are similar to those of the first embodiment.

DBDS and DBPC in insulating oil are gradually consumed due to use of the insulating oil in a transformer. Even in insulating oil having contained DBPC and DBDS before use, therefore, there is a case where DBDS and DBPC are not detected in a case of measuring only DBDS and DBPC after long-term use of the insulating oil. In such a case, there is a possibility that the transformer, which must be diagnosed as being at a high risk, is erroneously diagnosed as being at a low risk.

This embodiment can avoid such an erroneous determination by detecting a compound (reaction product of the radical resulting from DBDS) generated by consumption of DBDS in place of measurement of DBDS.

For example, benzaldehyde, benzyl alcohol, bibenzyl, dibenzyl (mono)sulfide and dibenzyl sulfoxide can be listed as reaction products of radicals resulting from DBDS. These compounds can be analyzed by employing gas chromatograph/mass spectrometry, for example.

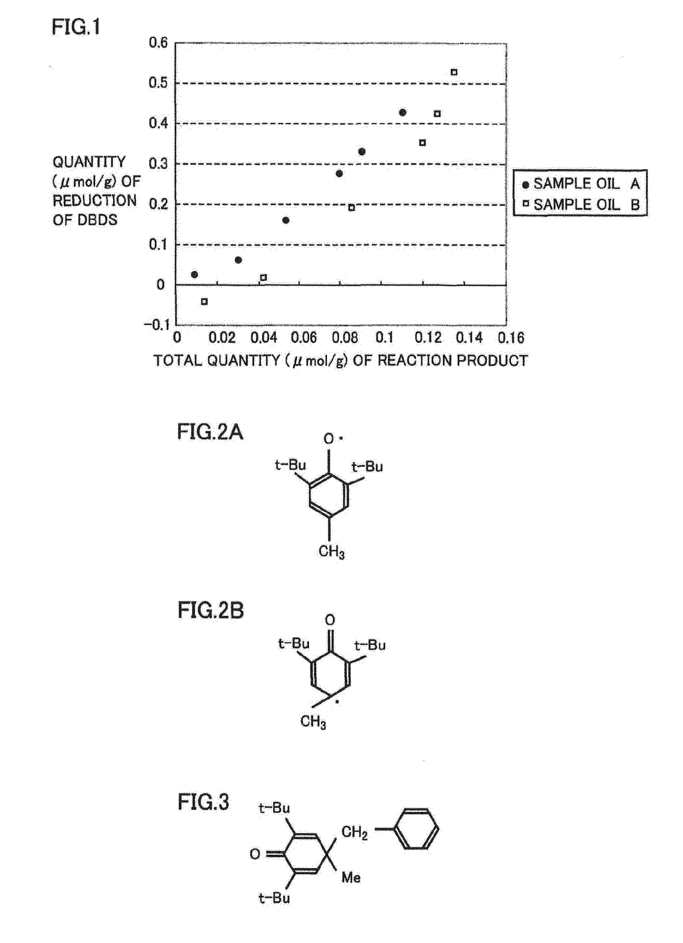

FIG. 1 shows the relation between the quantities of reduction (quantities of consumption) of DBDS and the total quantities of these reaction products (benzaldehyde, benzyl alcohol, bibenzyl, dibenzyl (mono)sulfide and dibenzyl sulfoxide) in sample oil A and sample oil B in Test Example 1 described later.

As shown in FIG. 1, the total quantities of these reaction products and the quantities of reduction of benzyl sulfide are correlated with each other. Therefore, it is determinable that DBDS has been added if these compounds are detected also in a case where DBDS is not detected.

These compounds are correlated with the quantity of reduction of DBDS also as individual contents, and hence it is provable that DBDS has been present without employing the total quantity, by measuring at least one of these compounds.

As to detection of DBPC, whether or not DBPC has been added to electric insulating oil at the time of start of use can be determined by a method of analyzing a reaction product of DBPC and a peroxy radical by a proper analytical method with a gas chromatograph mass spectrometer, for example. It is widely known that DBPC easily becomes a radical shown in FIG. 2A or 2B and reacts with a radical such as a peroxy radical.

### Third Embodiment

This embodiment is a diagnosis method different from the first and second embodiments in a point of measuring a reaction product of a radical resulting from DBPC and a radical resulting from DBDS as the aforementioned specific compound, while the remaining points are similar to those of the first and second embodiments.

The measurement of the reaction product of the radical resulting from DBPC and the radical resulting from DBDS corresponds to measurement of "a specific compound containing a reaction product of a radical resulting from dibenzyl disulfide and a reaction product of a radical resulting from di-tert-butyl-p-cresol" in the diagnosis method according to the present invention.

In other words, the detection of "the specific compound containing the reaction product of the radical resulting from dibenzyl disulfide and the reaction product of the radical resulting from di-tert-butyl-p-cresol" includes not only a case of detecting both of a reaction product of radicals resulting from dibenzyl disulfide and a reaction product of radicals resulting from di-tert-butyl-p-cresol, but also a case of detecting a reaction product of a radical resulting from dibenzyl disulfide and a radical resulting from di-tert-butyl-p-cresol.

When analyzing electric insulating oil with a gas chromatograph/mass spectrometer (GC/MS) in a case where DBPC and DBDS have been added at the same time, a compound whose molecular weight is 310 is detected. It is conceivable that this compound whose molecular weight is 310 is 4-benzyl-2,6-di-tert-butyl-4-methyl-2,5-cyclohexadienone (whose structural formula is shown in FIG. 3), for example.

This compound is a reaction product of a radical resulting from DBPC. and a benzyl radical which is a radical resulting from DBDS. With only detection of this compound, a risk of generation of copper sulfide in an oil-filled electrical apparatus can be evaluated similarly to the first embodiment detecting DBPC and DBDS.

According to the diagnosis method of this embodiment, it is determinable that there is a high risk that copper sulfide adheres to insulating paper in an oil-filled electrical apparatus similarly to the first embodiment, also when DBDS and DBPC have been consumed due to generation of copper sulfide or the like as a result of long-term use of insulating oil. Further, the oil-filled electrical apparatus can be simply diagnosed with only measurement of one type of compound.

In the diagnosis method according to the present invention, a risk of copper sulfide generation in an oil-filled electrical apparatus may be evaluated by detecting DBDS and DBPC in insulating oil and further detecting a reaction product, such as 4-benzyl-2,6-di-tert-butyl-4-methyl-2,5-cyclohexadienone, of a radical resulting from DBDS and a radical resulting from DBPC, for example. In this case, the concentrations of DBDS and DBPC in the insulating oil before use can be more correctly estimated, whereby the risk of copper sulfide generation on an insulating paper surface in the oil-filled electrical apparatus can be more correctly evaluated.

Also in a case of regarding DBP as a detection object in place of DBPC in the aforementioned first to third embodiments, it is possible to correctly evaluate a risk of copper sulfide generation on an insulating paper surface of a coil or the like dipped in insulating oil in an oil-filled electrical apparatus.

### Fourth Embodiment

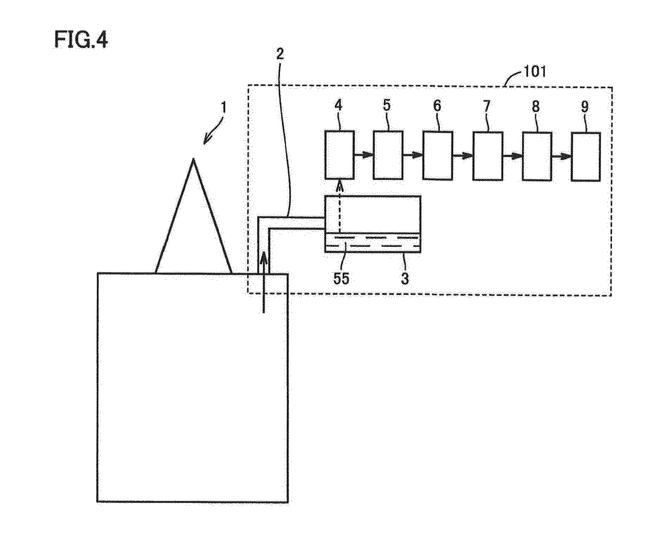

This embodiment is an embodiment of a diagnosis method for implementing the diagnosis methods for oil-filled electrical apparatuses according to the aforementioned embodiments. FIG. 4 shows a block diagram of a diagnosis apparatus for an oil-filled electrical apparatus according to this embodiment. Referring to FIG. 4, a diagnosis apparatus 101 includes a pipe 2, a tank 3, an oil collector 4, a pretreater 5, a detection unit 6, an evaluation unit 7, a diagnosis unit 8 and a display 9.

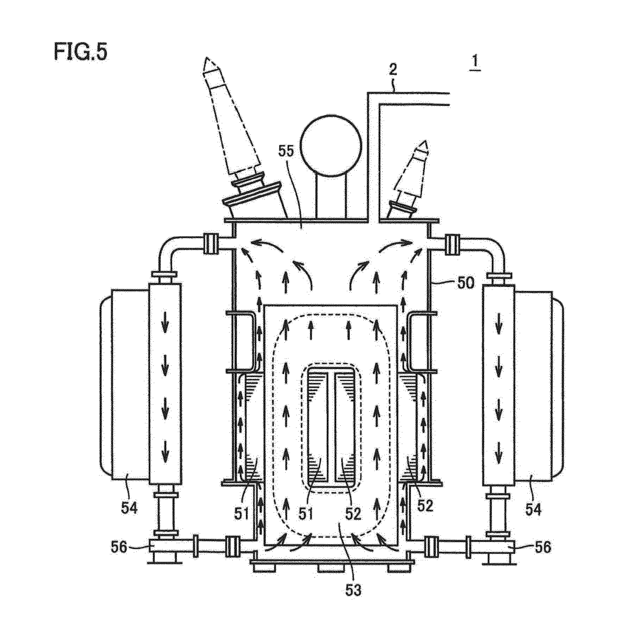

FIG. 5 is a sectional view showing a structural example of an oil-filled electrical apparatus shown in FIG. 4. Referring to FIG. 5, an oil-filled electrical apparatus 1 is a transformer, for example, and includes a tank 50, iron cores 51 and 52, a coil 53, coolers 54 and insulating oil 55.

The iron cores 51 and 52 and the coil 53 are stored in the tank 50. The coil 53 is enclosed by the iron cores 51 and 52. The inner portion of the tank 50 is filled with the insulating oil 55. Therefore, the coil 53 is dipped in insulating oil 55.

Insulating oil 55 circulates in the oil-filled electrical apparatus 1 by pumps 56. As shown by arrows in FIG. 5, the insulating oil 55 goes out of the tank 50, and is cooled by the coolers 54. The cooled insulating oil 55 returns to the tank 50. The insulating oil 55 is a mineral oil or a synthetic oil, for example.

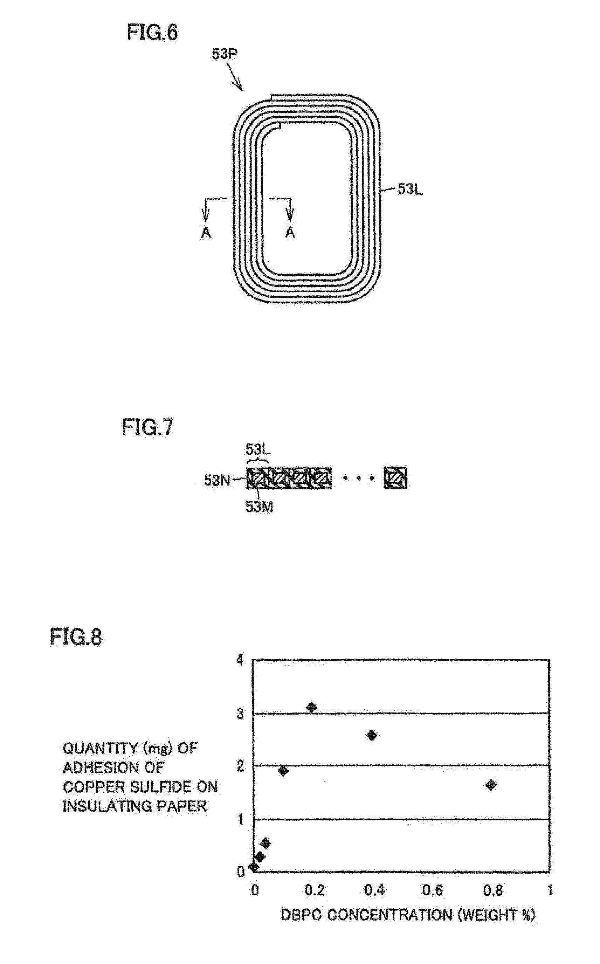

The coil 53 is constituted of a plurality of winding layers stacked in one direction. FIG. 6 is a plan view showing one of the plurality of winding layers constituting the coil. FIG. 7 is a sectional view showing a section of the winding layer shown in FIG. 6 along the line A-A.

Referring to Figs. 6 and 7, a winding layer 53P is constituted of a paper-wrapped conductor 53L. The paper-wrapped conductor 53L is spirally wound in the same plane. The paper-wrapped conductor 53L has a conductor 53M containing copper and insulating paper 53N covering the conductor 53M. The insulating paper 53N contains cellulose molecules.

Referring to FIG. 4, the tank 3 is connected to the oil-filled electrical apparatus I by the pipe 2. When the insulating oil 55 is collected from inside oil-filled electrical apparatus 1, a part of the insulating oil in the oil-filled electrical apparatus I passes through the pipe 2, and flows into the tank 3. The oil collector 4 is a pump, for example, and collects the insulating oil in the tank 3. The insulating oil in the tank 3 is employed for detection of specific compounds with the detection unit 6. The pretreater 5 performs pretreatment of the insulating oil before the insulating oil in the tank 3 is transmitted to the detection unit 6. In the detection unit 6, residual concentrations of the aforementioned specific compounds are measured.

The evaluation unit 7 and the diagnosis unit 8 are constituted of computers, for example, and execute arithmetic processing on the basis of maps and programs stored therein. More specifically, the evaluation unit 7 receives measured values of the specific compounds from the detection unit 6, and evaluates a possibility of copper sulfide generation in the oil-filled electrical apparatus. The diagnosis unit 8 receives the result of evaluation from the evaluation unit 7, and diagnoses a risk of anomaly occurrence in the oil-filled electrical apparatus 1.

The display 9 displays the result of diagnosis by the diagnosis unit 8, i.e., the risk of anomaly occurrence in the oil-filled electrical apparatus on a screen (not shown). Thus, it becomes possible to grasp the result of diagnosis by the diagnosis apparatus 101.

### EXAMPLES

Results of a test of confirming the relation between the quantities of compounds contained in insulating oil deriving from dibenzyl disulfide; and di-tert-butyl-p-cresol or di-tert-butyl-phenol, and generation of copper sulfide on insulating paper surfaces are now shown in relation to the diagnosis method according to the present invention.

### Test Example I

First, naphthene-based transformer oil (sample oil A) confirmed as containing no corrosive sulfur under ASTM D 1275B is prepared. Then, dibenzyl disulfide is added to this transformer oil by a prescribed quantity. The same was added by 100 ppmw (w/w) in this experiment. This oil is regarded as sample oil B. Oil prepared by adding DBPC to the sample oil B by 0.4 weight % (w/w) is regarded as sample oil C.

A test related to generation of copper sulfide is conducted by a method according to IEC 62535 of IEC (International Electrotechnical Commission) standards by employing these insulating oil samples. 15 grams of this transformer oil and a copper plate (30 mm by 7.5 mm by 1.5 mm) wrapped with one layer of kraft paper are sealed in a bottle having an internal volume of 30 cm³, covered with a silicone rubber stopper, and thereafter heated under conditions of 150 °C and 24 to 144 hours.

Table 1 shows the relation between copper sulfide adhesion situations on insulating paper surfaces after the test and heating times. The numerical values in the table are employed in the following meanings:
1 = no adhesion of copper sulfide
2 = slightly adhered to end portion of insulating paper
3 = adhered in wider range than 2
4 = adhered to whole surface

**Table 1**

| | 1^{st} Day | 2^{nd} Day | 3^{rd} Day | 4^{th} Day | 5^{th} Day | 6^{th} Day |
|---|---|---|---|---|---|---|
| Sample Oil A | 1 | 1 | 1 | 1 | 1 | 1 |
| Sample Oil B | 1 | 2 | 2 | 2 | 2 | 2 |
| Sample Oil C | 3 | 3 | 3 | 3 | 4 | 4 |

As shown in Table 1, no dibenzyl sulfide serving as the raw material for copper sulfide is added and hence no copper sulfide is generated in sample oil A. In sample oil B, generation of copper sulfide on an insulating paper surface is observed. As a result of obtaining the quantity of adhesion of copper sulfide by analysis, the same was 1.9 (1^{st} day) to 9.5 µg/cm² (6^{th} day). In sample oil C, remarkable generation of copper sulfide on an insulating paper surface is observed. As a result of obtaining the quantity of adhesion of copper sulfide by analysis, the same was 48 (1^{st} day) to 47.7 µg/cm² (6^{th} day).

Therefore, a case of employing sample oil C can be regarded as being at a high risk of at least about five times as compared with a case of employing sample oil B. In other words, a transformer employing insulating oil to which DBDS and DBPC are added can be determined as being at a high risk (having a high risk of anomaly occurrence).

### Test Example 2

The following investigation was conducted by the same method as Test Example 1, except that paraffin-based mineral oil was used in place of the naphthene-based mineral oil:

Paraffin-based transformer oil (sample oil D) confirmed as containing no corrosive sulfur under ASTM D 1275B is prepared. Then, oil prepared by adding dibenzyl disulfide to this transformer oil (sample oil D) by 100 ppmw is regarded as sample oil E. Oil prepared by adding DBPC to sample oil E by 0.4 weight % is regarded as sample oil F.

A test related to generation of copper sulfide was conducted by employing these insulating oil samples by a method according to IEC 62535. 15 grams of this transformer oil and a copper plate (30 mm by 7.5 mm by 1.5 mm) wrapped with one layer of kraft paper are sealed in a bottle having an internal volume of 30 cm³, covered with a silicone rubber stopper, and thereafter heated under conditions of 150 °C and 24 to 144 hours.

Table 2 shows the relation between copper sulfide adhesion situations on insulating paper surfaces after the test and heating times. The numerical values in the table are employed in meanings similar to those in Table 1.

**Table 2**

| | 1^{st} Day | 2^{nd} Day | 3^{rd} Day | 4^{th} Day | 5^{th} Day | 6^{th} Day |
|---|---|---|---|---|---|---|
| Sample Oil D | 1 | 1 | 1 | 1 | 1 | 1 |
| Sample Oil E | 2 | 2 | 2 | 2 | 2 | 2 |
| Sample Oil F | 1 | 3 | 3 | 3 | 4 | 4 |

As shown in Table 2, no dibenzyl disulfide serving as the raw material for copper sulfide is added and hence no copper sulfide is generated in sample oil D. In sample oil E, generation of copper sulfide on an insulating paper surface is observed. As a result of analyzing the quantity of adhesion of copper sulfide, the quantity of adhesion was 3.5 µg/cm² (1^{st} day) to 10.7 µg/cm² (6^{th} day).

In sample oil F, a remarkable generation of copper sulfide on an insulating paper surface is observed. As a result of analyzing the quantity of adhesion of copper sulfide, the quantity of adhesion was 4.8 µg/cm² (1^{st} day) to 53.1 µg/cm² (6^{th} day).

Therefore, a case of employing sample oil F can be regarded as being at a high risk of about five times with respect to a case of employing sample oil D. In other words, a transformer employing insulating oil to which DBDS and DBPC are added can be determined as being at a high risk.

### Test Example 3

In Test Examples 1 and 2, the experiments were conducted while fixing the concentrations of DBPC to 0.4 weight %. In this Test Example, a test related to generation of copper sulfide similar to that in Test Example 1 was conducted while setting concentrations of DBPC to 0, 0.02, 0.04, 0.1, 0.2, 0.4 and 0.8 weight % and setting concentrations of DBDS to 100 ppmw in order to clarify the influence exerted by the concentrations of DBPC on quantities of copper sulfide on insulating paper surfaces, and quantities of adhesion of copper sulfide on insulating paper were measured. FIG. 8 shows a graph plotting the quantities of adhesion of copper sulfide on insulating paper with respect to the concentrations of DBPC.

As shown in FIG. 8, the quantity of adhesion of copper sulfide on insulating paper clearly increases as compared with an unadded case, even if the concentration of DBPC is 0.02 weight %. The quantities of copper sulfide on the insulating paper surfaces increased following the increase of the DBPC concentrations up to the DBPC loading of 0.2 weight %, and the quantities of copper sulfide on insulating paper thereafter decreased from the maximum value of 0.2 weight % following the increase of the concentrations of DBPC.

### Test Example 4

In Test Examples 1 to 3, the experiments employing DBPC as antioxidants were conducted. In this Test Example, an experiment employing DBP (di-tert-butyl-phenol) as antioxidants was conducted. In other words, an experiment similar to that in Test Example 1 was conducted as to naphthene-based transformer oil (sample oil A) similar to that in Test Example 1 and oil (sample oil G) prepared by adding 0.4 weight % of DBP and 100 ppmw of DBDS to sample oil A.

Table 3 shows the relation between copper sulfide adhesion situations on insulating paper surfaces after the test and heating times. Numerical values in the table are employed in meanings similar to those in Table 1.

**Table 3**

| | 1^{st} Day | 2^{nd} Day | 3^{rd} Day | 4^{th} Day | 5^{th} Day | 6^{th} Day |
|---|---|---|---|---|---|---|
| Sample Oil A | 1 | 1 | 1 | 1 | 1 | 1 |
| Sample Oil G | 3 | 3 | 3 | 3 | 3 | 3 |

As shown in Table 3, the quantities of copper sulfide adhering to the insulating paper surfaces were small not to reach full-scale adhesion in the case of DBP as compared with the case of DBPC, while the quantities of adhesion of copper sulfide on the insulating paper surfaces clearly increased as compared with the case (sample oil B in Table 1) where no antioxidant was added. Therefore, a transformer employing insulating oil to which DBP is added along with DBDS can be determined as being at a higher risk than a transformer employing insulating oil to which neither DBP nor DBPC is added.

The embodiments and Examples disclosed this time must be considered as illustrative in all points and not restrictive. The range of the present invention is shown not by the above description but the scope of claims for patent, and it is intended that all modifications within the meaning and range equivalent to the scope of claims for patent are included.

### LIST OF PREFERENCE SIGNS

- 1: = oil-filled electrical apparatus
- 2: = pipe
- 3: = tank
- 4: = oil collector
- 5: = pretreater
- 6: = detection unit
- 7: = evaluation unit
- 8: = diagnosis unit
- 9: = display
- 50: = tank
- 51: = iron core
- 52: = iron core
- 53: = coil
- 53L: = paper-wrapped conductor
- 53M: = conductor
- 53N: = insulating paper
- 53P: = winding layer
- 54: = cooler
- 55: = insulating oil
- 56: = pump
- 101: = diagnosis apparatus.

## Claims

1. A diagnosis method for an oil-filled electrical apparatus (1) evaluating a risk of copper sulfide generation in the oil-filled electrical apparatus (1), comprising:
- a first step of detecting a specific compound contained in insulating oil (55) in the oil-filled electrical apparatus (1);
- a second step of evaluating a possibility of copper sulfide generation in the oil-filled electrical apparatus (1) on the basis of the result of detection obtained in the first step; and
- a third step of diagnosing a risk of anomaly occurrence in the oil-filled electrical apparatus (1) on the basis of the result of evaluation obtained in the second step, wherein
- the specific compound contains dibenzyl disulfide and/or a reaction product of a radical resulting from dibenzyl disulfide, and
- di-tert-butyl-p-cresol and/of a reaction product of a radical resulting from di-tert-butyl-p-cresol, or, di-tert-butyl-phenol and/or a reaction product of a radical resulting from di-tert-butyl-phenol.

2. The diagnosis method for an oil-filled electrical apparatus (1) according to claim 1,
wherein the specific compound contains dibenzyl sulfide, and di-tert-butyl-p-cresol or di-tert-butyt-phenol.

3. The diagnosis method for an oil-filled electrical apparatus (1) according to claim 1,
wherein the reaction product of the radical resulting from the dibenzyl disulfide is a compound of at least one type selected from a group consisting of benzaldehyde, benzyl alcohol, bibenzyl, dibenzyl sulfide and dibenzyl sulfoxide.

4. The diagnosis method for an oil-filled electrical apparatus (1) according to claim 1,
wherein the specific compound contains a reaction product of a radical resulting from dibenzyl disulfide and a radical resulting from di-tert-butyl-p-cresol, or, a reaction product of a radical resulting from Dibenzyl disulfide and a radical resulting from di-tert-butyl-phenol.

5. The diagnosis method for an oil-filled electrical apparatus. (1) according to claim 1,
evaluating in the second step that the possibility of copper sulfide generation in the oil-filled electrical apparatus (1) is high in a case where the specific compound is detected in the first step, and
diagnosing in the third step that the risk of anomaly occurrence in the oil-filled electrical apparatus (1) is high in a case where the possibility of copper sulfide generation is evaluated as high in the second step.

6. The diagnosis method for an oil-filled electrical apparatus (1) according to claim 1,
wherein copper sulfide generation in the oil-filled electrical apparatus (1) is copper sulfide generation on a surface of insulating paper (53N).

7. A diagnosis apparatus (101) for an oil-filled electrical apparatus (1) for evaluating a risk of copper sulfide generation in the oil-filled electrical apparatus (1), comprising:
- a detection unit (6) detecting a specific compound contained in insulating oil (55) in the oil-filled electrical apparatus (1);
- an evaluation unit (7) evaluating a possibility of copper sulfide generation in the oil-filled electrical apparatus (1) on the basis of the result of detection obtained in the detection unit (6); and
- a diagnosis unit (6) diagnosing a risk of anomaly occurrence in the oil-filled electrical apparatus, (1) on the basis of the result of evaluation obtained in the evaluation unit (7), wherein
- the specific compound contains dibenzyl disulfide and/or a reaction product of a radical resulting from dibenzyl disulfide, and
- di-tert-butyl-p-cresol and/or a reaction product of a radical resulting from di-tert-butyl-p-cresol, or, di-tert-butyl-phenol and/or a reaction product of a radical resulting from di-tert-butyl-phenol.
